(19) Europäisches Patentamt
European Patent Office
Office européen des brevets

(11) Numéro de publication : **0 302 799 B1**

# (12) FASCICULE DE BREVET EUROPEEN

(45) Date de publication du fascicule du brevet : **09.10.91 Bulletin 91/41**

(51) Int. Cl.[5] : **C07C 271/10**

(21) Numéro de dépôt : **88402050.4**

(22) Date de dépôt : **05.08.88**

## (54) Procédé de préparation de l'acide N6-benzyloxycarbonyl diamino-2,6 pimélamique sous les formes L,L ou D,D ou racémique.

(30) Priorité : **07.08.87 FR 8711257**

(43) Date de publication de la demande : **08.02.89 Bulletin 89/06**

(45) Mention de la délivrance du brevet : **09.10.91 Bulletin 91/41**

(84) Etats contractants désignés : **AT BE CH DE FR GB IT LI LU NL SE**

(56) Documents cités : **FR-A- 2 460 289**

(73) Titulaire : **RHONE-POULENC SANTE
20, avenue Raymond Aron
F-92160 Antony (FR)**

(72) Inventeur : **Bouchaudon, Jean
23, avenue des Saules
F-91390 Morsang sur Orge (FR)**
Inventeur : **Farge, Daniel
30, rue des Pins Sylvestres
F-94320 Thiais (FR)**
Inventeur : **James, Claude
31, bis, avenue Gammbetta
F-75020 Paris (FR)**

(74) Mandataire : **Pilard, Jacques et al
RHONE-POULENC INTERSERVICES Service
Brevets Pharma 25, Quai Paul Doumer
F-92408 Courbevoie Cédex (FR)**

**EP 0 302 799 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé de préparation de l'acide N⁶-benzyloxycarbonyl diamino-2,6 pimélamique de formule :

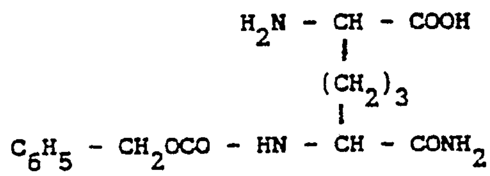

(I)

sous les formes L,L ou D,D ou racémique.

Le produit de formule (I) est un intermédiaire de synthèse des peptides qui font l'objet des brevets français FR 7916844 (2460289), FR 7916845 (2460290), FR 8011231 (2482958) et FR 8011233 (2482960) et qui présentent des propriétés immunostimulantes remarquables.

Selon le brevet français FR 7916844 (2460289), le produit de formule (I) peut être obtenu à partir de l'acide diamino-2,6 pimélique. A cet effet, on prépare selon les méthodes connues l'ester dibenzylique de l'acide dibenzyloxycarbonylamino-2,6 pimélique qui est mono-saponifié selon la méthode décrite par A. ARENDT et coll., Roczniki Chemii Ann. Soc. Chim. Polonorum, 48, 1305 (1974) [Chem. Abstr., 82, 31497 g (1975)] puis transformé, par action du méthanol ammoniacal, en monoacide de formule :

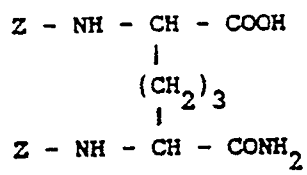

(II)

dans laquelle Z représente un radical benzyloxycarbonyle qui, après hydrogénolyse en présence de palladium sur noir, fournit l'acide diamino-2,6 pimélamique.

Par action d'un sel de cuivre, tel que le bromure cuivrique ou le carbonate basique de cuivre, sur l'acide diamino-2,6 pimélamique, il se forme un complexe qui peut être représenté par la formule :

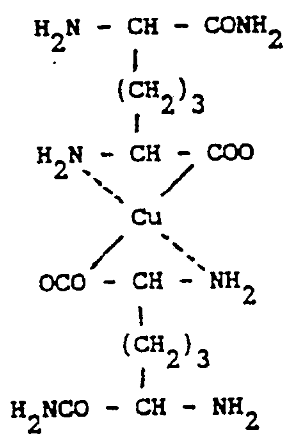

(III)

dont les fonctions amines libres sont protégées par action d'un halogénoformiate de benzyle. Le complexe ainsi formé est déplacé par action de l'hydrogène sulfuré pour donner le produit de formule (I).

A partir de l'acide dibenzyloxycarbonylamino-2,6 pimélique, l'acide N⁶-benzyloxycarbonyl diamino-2,6 pimélamique est obtenu avec un rendement global voisin de 9 %.

Il a maintenant été trouvé, et c'est ce qui fait l'objet de la présente invention, que l'acide N⁶-benzyloxycarbonyl diamino-2,6 pimélamique de formule (I) peut être obtenu à partir de l'acide dibenzyloxycarbonylamino-2,6 pimélique avec un rendement nettement amélioré par un procédé comportant un plus petit nombre d'étapes.

Selon la présente invention, l'acide N⁶-benzyloxycarbonyl diamino-2,6 pimélamique peut être obtenu par

action d'un agent d'amidification sur l'acide $O^1$-p.nitrobenzyl $N^2$-benzyloxycarbonyl diamino-2,6 pimélique. Généralement, l'amidification est réalisée à une température voisine de 20°C au moyen d'une solution d'ammoniaque en opérant en présence d'un solvant non miscible à l'eau choisi parmi les hydrocarbures aromatiques (toluène), les hydrocarbures aliphatiques halogénés (chlorure de méthylène), les éthers (éther éthylique) ou les esters (acétate d'éthyle).

L'acide $O^1$-p.nitrobenzyl $N^2$-benzyloxycarbonyl diamino-2,6 pimélique peut être obtenu par action d'un agent capable de transformer un acide en l'halogénure d'acyle correspondant, tel que le pentachlorure de phosphore, le tribromure de phosphore ou le chlorure de thionyle, sur l'acide $O^1$-p.nitrobenzyl dibenzyloxycarbonylamino-2,6 pimélique.

De préférence, on utilise le pentachlorure de phosphore en opérant dans un solvant organique tel que le chlorure de méthylène à une température comprise entre 0 et 30°C.

L'acide $O^1$-p.nitrobenzyl dibenzyloxycarbonylamino-2,6 pimélique peut être préparé à partir de l'acide diamino-2,6 pimélique selon le procédé décrit par A. ARENDT et coll., Roczniki Chemii Ann. Soc. Chim. Polonorum, 48, 1501 (1974) [Chem. Abstr., 82, 171409 n(1975)].

Le procédé selon la présente invention permet, à partir d'un acide $O^1$-p.nitrobenzyl dibenzyloxycarbonylamino-2,6 pimélique sous forme L,L ou D,D ou racémique, d'obtenir l'acide $N^6$-benzyloxycarbonyl diamino-2,6 pimélamique respectivement sous forme L,L ou D,D ou racémique.

A partir de l'acide dibenzyloxycarbonylamino-2,6 pimélique, l'acide $N^6$-benzyloxycarbonyl diamino-2,6 pimélamique est obtenu, selon le procédé de la présente invention, avec un rendement global généralement supérieur à 20 %.

Les exemples suivants, donnés à titre non limitatif, montrent comment l'invention peut être mise en pratique.

## EXEMPLE 1

A un mélange de 1,24 l d'ammoniaque à 20 % et de 175 $cm^3$ d'acétate d'éthyle, on ajoute 57,9 g d'acide $O^1$-p.nitrobenzyl $N^2$-benzyloxycarbonyl L,L diamino-2,6 pimélique. On agite pendant 24 heures à 20°C environ. La phase aqueuse est séparée par décantation et est lavée 3 fois avec 600 $cm^3$ au total d'acétate d'éthyle, puis est refroidie vers 10°C et enfin est neutralisée à pH=6 par addition de 250 $cm^3$ d'acide acétique anhydre. On obtient une bouillie que l'on agite pendant 1/2 heure vers 5°C. On filtre, lave l'insoluble 2 fois par 400 $cm^3$ au total d'acide acétique et 2 fois par 400 $cm^3$ au total d'acétate d'éthyle. Après séchage sous pression réduite (20 mm de mercure ; 2,7 kPa), on obtient 23,5 g d'acide $N^6$-benzyloxycarbonyl L,L diamino-2,6 pimélamique dont les caractéristiques sont les suivantes :

— Rf = 0,46 [silicagel ; n.butanol-pyridine-acide acétique-eau (50-20-6-24 en volumes)],

— Rf = 0,44 [silicagel ; acétate d'éthyle-acide acétique-eau (40-12-10 en volumes)],

— après hydrolyse méthylante (méthanol chlorhydrique 6N à ébullition) suivie d'une acylation avec l'anhydride trifluoracétique, le dosage par chromatographie en phase gazeuse sur colonne CHIRASIL-VAL montre que le produit obtenu contient 99,8 % de forme L,L et 0,2 % de forme méso.

— $[\alpha]_D^{20}$ = + 18,2° (acide acétique, c = 0,3)

L'acide $O^1$-p.nitrobenzyl $N^2$-benzyloxycarbonyl L,L diamino-2,6 pimélique peut être préparé de la façon suivante :

A une solution refroidie à 5°C de 268 g d'acide L,L $O^1$-p.nitrobenzyl dibenzyloxycarbonylamino-2,6 pimélique dans 4 l de chlorure de méthylène, on ajoute en 15 minutes, par petites portions, 112,8 g de pentachlorure de phosphore. On agite le milieu réactionnel pendant 50 minutes à 5°C, puis pendant 15 minutes à 20°C. Ensuite, en 50 minutes, on élimine du milieu réactionnel 2 l de chlorure de méthylène par distillation à pression ordinaire. Au concentrat refroidi à 5°C, on ajoute 6,66 l d'éther de pétrole. On abandonne à 5°C pendant 24 heures puis on élimine la phase éthérée. L'huile résiduelle est triturée 3 fois avec 1,5 l au total d'éther de pétrole puis est dissoute dans 1 l de chlorure de méthylène. Après concentration à sec sous pression réduite (20 mm de mercure ; 2,7 kPa) à 50°C, la meringue obtenue est dissoute dans 1,74 l d'acide acétique anhydre. A cette solution, on ajoute 0,87 l d'eau. On abandonne à 20°C pendant 24 heures, puis on verse la solution dans un mélange de 7,2 l d'eau et de 2 l d'éther. On amène la phase aqueuse à pH=5 par addition lente de 400 g de carbonate de sodium. Le précipité formé est séparé par filtration, lavé successivement 2 fois par 1 l au total d'eau distillée et 3 fois par 1,5 l au total d'éther. Après séchage à l'air libre, puis sous pression réduite (20 mm de mercure ; 2,7 kPa) à 50°C, on obtient 169,3 g d'acide $O^1$-p.nitrobenzyl $N^2$-benzyloxycarbonyl L,L diamino-2,6 pimélique dont les caractéristiques sont les suivantes :

— Rf = 0,61 [silicagel ; n.butanol-pyridine-acide acétique-eau (50-20-6-24 en volumes)].

L'acide L,L 01-p.nitrobenzyl dibenzyloxycarbonylamino-2,6 pimélique peut être préparé selon la méthode

de A. ARENDT et coll., Roczniki Chemii Ann. Soc. Chim. Polonorum, 48, 1501 (1974).

EXEMPLE 2

A un mélange de 226 $cm^3$ d'ammoniaque à 20 % et de 35 $cm^3$ d'acétate d'éthyle, on ajoute 10,4 g d'acide $O^1$-p.nitrobenzyl $N^2$-benzyloxycarbonyl D,D diamino-2,6 pimélique. On agite pendant 3 heures à une température voisine de 20°C. La phase aqueuse est séparée par décantation, lavée 3 fois avec 120 $cm^3$ au total d'acétate d'éthyle, concentrée partiellement jusqu'à pH=7 sous pression réduite (20 mm de mercure ; 2,7 kPa) à 50°C. On obtient ainsi une bouillie qui est refroidie à 4°C pendant 1 heure, filtrée et lavée 3 fois par 30 $cm^3$ au total d'eau. Après séchage sous pression réduite (20 mm de mercure ; 2,7 kPa) à 20°C, on obtient 5,36 g d'acide $N^6$-benzyloxycarbonyl D,D diamino-2,6 pimélamique dont les caractéristiques sont les suivantes :

— Rf = 0,46 [silicagel ; n.butanol-pyridine-acide acétique-eau (50-20-6-24 en volumes)].

L'acide $O^1$-p.nitrobenzyl $N^2$-benzyloxycarbonyl D,D diamino-2,6 pimélique peut être préparé de la même manière que l'isomère L,L.

Ainsi à partir de 26,57 g d'acide D,D $O^1$-p.nitrobenzyl dibenzyloxycarbonylamino-02,6 pimélique, de 11,18 g de pentachlorure de phosphore et de 460 $cm^3$ de chlorure de méthylène, on obtient 10,44 g d'acide $O^1$-p.nitrobenzyl $N^2$-benzyloxycarbonyl D,D diamino-2,6 pimélique dont les caractéristiques sont les suivantes :

— Rf = 0,61 [silicagel ; n.butanol-pyridine-acide acétique-eau (50-20-6-24 en volumes)].

L'acide D,D $O^1$-p.nitrobenzyl dibenzyloxycarbonylamino-2,6 pimélique peut être préparé selon la méthode de A. ARENDT et coll., Roczniki Chemii Ann. Soc. Chim. Polonorum, 48, 1501 (1974).

EXEMPLE 3

En opérant de la même manière que dans l'exemple 2, à partir de 52,38 g d'acide $O^1$-p.nitrobenzyl $N^2$-benzyloxycarbonyl DD,LL diamino-2,6 pimélique, de 1900 $cm^3$ d'ammoniaque à 20 % et de 270 $cm^3$ d'acétate déthyle, on obtient 26,58 g d'acide $N^6$-benzyloxycarbonyl DD,LL diamino-2,6 pimélamique dont les caractéristiques soint les suivantes :

— Rf = 0,46 [silicagel ; n.butanol-pyridine-acide acétique-eau (50-20-6-24 en volumes)].

L'acide $O^1$-p.nitrobenzyl $N^2$-benzyloxycarbonyl DD,LL diamino-2,6 pimélique peut être préparé de la même manière que l'isomère L,L.

Ainsi à partir de 114 g d'acide DD,LL $O^1$-p.nitrobenzyl dibenzyloxycarbonylamino-2,6 pimélique, de 48 g de pentachlorure de phosphore et de 1,5 l de chlorure de méthylène, on obtient 52,9 g d'acide $O^1$-p.nitrobenzyl $N^2$-benzyloxycarbonyl DD,LL diamino-2,6 pimélique dont les caractéristiques sont les suivantes :

— Rf = 0,61 [silicagel ; n.butanol-pyridine-acide acétique-eau (50-20-6-24 en volumes)].

L'acide DD,LL $O^1$-p.nitrobenzyl dibenzyloxycarbonylamino-2,6 pimélique peut être préparé selon la méthode de A. ARENDT et coll., Roczniki Chemii Ann. Soc. Chim. Polonorum, 48, 1501 (1974).

## Revendications

1. Procédé de préparation de l'acide $N^6$-benzyloxycarbonyl diamino-2,6 pimélamique sous forme L,L ou D,D ou racémique caractérisé en ce que l'on fait réagir un agent d'amidification sur l'acide $O^1$-p.nitrobenzyl $N^2$-benzyloxycarbonyl diamino-2,6 pimélique sous forme L,L ou D,D ou racémique qui peut lui-même être obtenu par action d'un agent capable de transformer un acide en l'halogénure d'acyle correspondant choisi parmi le pentachlorure de phosphore, le tribromure de phosphore et le chlorure de thionyle sur l'acid $O^1$-p.nitrobenzyl dibenzyloxycarbonylamino-2,6 pimélique sous forme L,L ou D,D ou racémique.

2. Procédé selon la revendication 1 caractérisé en ce que l'amidification est réalisée au moyen d'une solution aqueuse d'ammoniaque en opérant dans un solvant organique non miscible à l'eau choisi parmi les hydrocarbures aromatiques, les hydrocarbures aliphatiques halogénés, les éthers ou les esters.

3. Procédé selon la revendication 2 caractérisé en ce que le solvant est l'acétate d'éthyle.

## Patentansprüche

1. Verfahren zur Herstellung von $N^6$-Benzyloxycarbonyl-2,6-diamino-pimelaminsäure in L,L- oder D,D- oder racemischer Form, dadurch gekennzeichnet, daß man ein Amidifizierungsmittel auf $O^1$-p-Nitrobenzyl-$N^2$-benzyloxycarbonyl-2,6-diaminopimelinsäure in L,L- oder D,D- oder racemischer Form einwirken läßt, die ihrerseits durch Einwirkung eines Mittels, das eine Säure in das entsprechende Acylhalogenid umzuwandeln

vermag, ausgewählt aus Phosphorpentachlorid, Phosphortribromid und Thionylchlorid, auf $O^1$-p-Nitrobenzyl-2,6-dibenzyloxycarbonylaminopimelinsäure in L,L- oder D,D- oder racemischer Form erhalten werden kann.

2. Verfahren nach Anspruch 1, dadurch gekennzeichnet, daß die Amidifizierung mit Hilfe einer wässerigen Ammoniaklösung ausgeführt wird, indem man in einem mit Wasser nicht mischbaren organischen Lösungsmittel, ausgewählt aus den aromatischen Kohlenwasserstoffen, den halogenierten aliphatischen Kohlenwasserstoffen, den Äthern oder Estern, arbeitet.

3. Verfahren nach Anspruch 2, dadurch gekennzeichnet, daß das Lösungsmittel Äthylacetat ist.

## Claims

1. Process for the preparation of $N^6$-benzyloxycarbonyl-2,6-diaminopimelamic acid in L,L or D,D or racemic form, characterised in that an amidation agent is reacted with $O^1$-p-nitrobenzyl-$N^2$-benzyloxycarbonyl-2,6-diaminopimelic acid in L,L or D,D or racemic form, which may itself be obtained by the reaction of an agent capable of converting an acid into a corresponding acyl halide, chosen from phosphorus pentachloride, phosphorus tribromide and thionyl chloride, with $O^1$-p-nitrobenzyl-2,6-dibenzyloxycarbonylaminopimelic acid in L,L or D,D or racemic form.

2. Process according to Claim 1, characterised in that the amidation is carried out by means of an aqueous solution of aqueous ammonia by operating in a water-immiscible organic solvent chosen from aromatic hydrocarbons, halogenated aliphatic hydrocarbons, ethers or esters.

3. Process according to Claim 2, characterised in that the solvent is ethyl acetate.